# EUROPÄISCHE PATENTSCHRIFT

(11) **EP 0 486 882 B1**
(45) Veröffentlichungstag und Bekanntmachung des Hinweises auf die Patenterteilung: **31.01.1996**
(21) Anmeldenummer: 91118954.6
(22) Anmeldetag: 07.11.1991
(51) Int. Cl.: C07D 295/088, A61K 31/40, C07C 233/29, A61K 31/16

(54) **Substituierte Anilide**
Substituted anilides
Anilides substitués

(30) Priorität: 17.11.1990 DE 4036782
(43) Veröffentlichungstag der Anmeldung: 27.05.1992
(73) Patentinhaber: BASF Aktiengesellschaft, D-67063 Ludwigshafen (DE)
(72) Erfinder: Lubisch, Wilfried, Dr., W-6800 Mannheim 1 (DE); Von Philipsborn, Gerda, Dr., W-6940 Weinheim (DE); Schult, Sabine, Dr., W-6900 Heidelberg (DE); Kirchengast, Michael, Dr., W-6720 Speyer (DE)

(56) Entgegenhaltungen:
- EP-A- 0 352 639
- FR-A- 1 176 918
- US-A- 3 994 900
- JOURNAL OF MEDICINAL CHEMISTRY. Bd. 12, Nr. 1, Januar 1969, WASHINGTON US Seiten 164 - 166; J. KRAPCHO: 'IMMUNOSUPPRESIVE ACTIVITY OF 2'-(3-DIMETHYLAMINOPROPYLTHIO)CINNAMANILIDE (CINANSERIN) AND RELATED COMPOUNDS. IV'

## Beschreibung

Die vorliegende Erfindung betrifft neue substituierte Anilide der Formel I

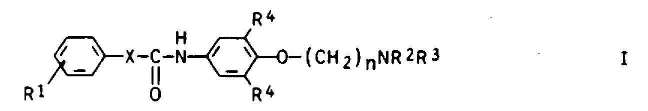

in der die Substituenten die folgende Bedeutung haben:
- R¹: C₁-C₄-Alkyl, C₁-C₄-Halogenalkyl, C₁-C₄-Alkoxy, Halogen, Cyano, Nitro, C₂-C₄-Alkenyl, C₂-C₄-Alkinyl;
- X: Methylen, Ethylen, Vinylen;
- R, R³: C₁-C₄-Alkyl, C₃-C₆-Cycloalkyl oder gemeinsam Tetra- oder Pentamethylen;
- R⁴: C₁-C₄-Alkyl, Methoxy;
- n: 2, 3 oder 4,
sowie deren physiologisch verträgliche Säureadditionssalze.

Weiterhin betrifft die Erfindung die Verbindungen I zur Verwendung auf dem Arzneimittelgebiet, die Verbindung I enthaltende Arzneimittel sowie die Herstellung der Verbindung I und deren Salze mit physiologisch verträglichen Säuren zur Herstellung von Arzneimitteln zur Behandlung von Herzrhythmusstörungen und/oder zur Herzfrequenzsenkung.

Arzneimittel zur Behandlung von Herzrhythmusstörungen (Antiarrythmika) teilt man aufgrund ihrer Wirkungsweise nach Vaughan-Williams (s. J. Clin. Pharmacol. 24 (1984) 129) in 4 Klassen ein:
A. Natrium-Antagonisten
B. Adrenerge β-Rezeptorblocker
C. Calcium-Antagonisten und
D. Repolarisationshemmstoffe.

Zu den Wirkstoffen der Klasse D zählen das Amiodaron (2-Butyl-3-benzofuranyl)-[4-[2-diethylamino)ethoxy]-3,5-diiodophenyl]keton) (s. Circulation 68 (1983) 88) und das D-Sotalol (4'-[1-Hydroxy-2(isopropylamino)-ethyl]methansulfonanilid) (s. Am. Heart. J. 109 (1985) 949). Sie zeichnen sich gegenüber Antiarrhythmika der anderen Klassen dadurch aus, daß sie gegen häufig therapieresistente Arrhythmien wie wiederkehrende Kammertachykardie und Kammerflimmern wirksam sind.

In J. Med. Chem. 12 (1969) 164 sind immunsuppressiv wirkende Zimtsäureanilide beschrieben, die wie die Verbindung II 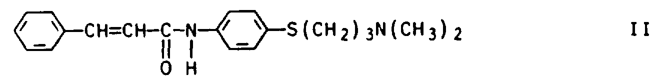 in der 4'-Position oder auch in der 2'-Position des Anilidrestes substituiert sind. Sie unterscheiden sich von den eingangs definierten Verbindungen I u.a. dadurch, daß sie nicht zusätzlich in der 3'- und 5'-Position substituiert sind.

Der Erfindung lag die Aufgabe zugrunde, neue und hochwirksame Antiarrhythmika, die als Repolarisationshemmstoffe wirken, bereitzustellen.

Demgemäß wurden die eingangs definierten Verbindungen I gefunden.

Außerdem wurden die Verwendung der Verbindungen I auf dem Arzneimittelgebiet, die Verbindungen I enthaltende Arzneimittel sowie die Verwendung der Verbindung I zur Herstellung von Arzneimitteln gefunden.

Neben ihrer Wirkung als Antiarrythmika, hat man bei den Aniliden I auch eine die Herzfrequenz senkende Wirkung gefunden.

Im Hinblick auf die bestimmungsgemäße Verwendung der Anilide I als Antiarrhythmika und die Herzfrequenz senkende Mittel kommen als Substituenten vorzugsweise folgende Reste in Betracht:
- R¹: C₁-C₄-Alkyl, wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl;
C₁-C₄-Halogenalkyl, vorzugsweise C₁-C₂-Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl;
C₁-C₄-Alkoxy wie Methoxy, Ethoxy, Propoxy, 1-Methylethoxy, Butoxy;
Halogen wie Fluor und Chlor;
Cyano; Nitro;
C₂-C₄-Alkenyl wie Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl; C₂-C₄-Alkinyl wie Ethinyl, 1-Propinyl, 2-Propinyl, 1-Butinyl, 2-Butinyl, 3-Butinyl, 1-Methyl-2-propinyl, insbesondere Ethinyl;
- X: Methylen, Ethylen, Vinylen;
- R, R³: C₁-C₄-Alkyl, wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl;
C₃-C₆-Cycloalkyl wie Cyclopropyl, Cyclopentyl und Cyclohexyl, oder gemeinsam Tetra- oder Pentamethylen;
- R⁴: C₁-C₄-Alkyl wie unter R¹ genannt, insbesondere Methyl, Ethyl, Propyl, tert.-Butyl, besonders bevorzugt Methyl, tert.-Butyl; Methoxy;
- n: 2, 3 oder 4.

Man erhält die Verbindungen I beispielsweise, indem man zunächst nach bekannten Methoden (s. u.a. Houben-Weyl, Band 6/3, Kap. A II, 1976) das Phenolderivat III 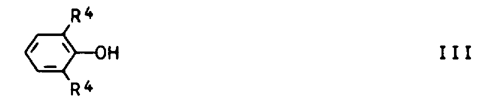 mit einem Amin L-(CH₂)ₙ-NRR³, in dem L eine nucleofuge Abgangsgruppe wie Chlor, Brom oder Tosylat bedeutet, zur Verbindung IV 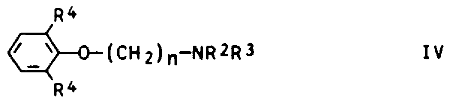 umsetzt, und diese dann in üblicher Weise, beispielsweise mit Salpetersäure, in der 4-Position zur Nitroverbindung V 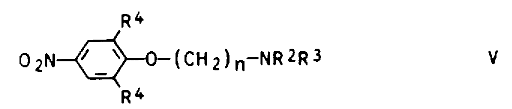 nitriert.

Eine andere Methode die Verbindung V herzustellen, besteht darin, daß man analog zu obigem Verfahren die in 4-Position nitrierte Phenolverbindung III' 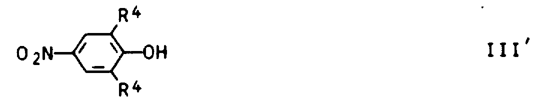 mit dem Amin L-(CH₂)ₙ-NRR³ umsetzt. Die Verbindungen III' sind bekannt oder lassen sich nach bekannten Methoden herstellen (s. Houben-Weyl, Bd. 10/1; S. 576 ff., 1971). Die Nitroverbindung V reduziert man dann in an sich bekannter Weise (s. Houben-Weyl Bd. XI/1, Seite 341 ff., 1957) zur Anilinverbindung VI. 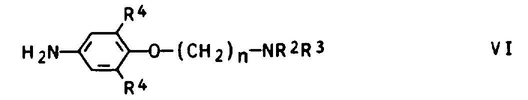 Anschließend setzt man das Anilinderivat VI in üblicher Weise (s. Houben-Weyl, Bd. E5, Kap. V, 1985) mit der in aktivierter Form vorliegenden Säure VII 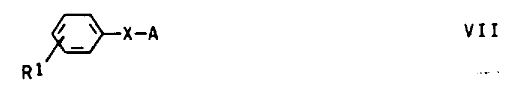 in der A Chloroformyl, C₁-C₃-Alkoxycarbonyl wie Methoxy-, Ethoxy- und Propoxycarbonyl, C₂-C₄-Acyloxycarbonyl, insbesonders Acetoxycarbonyl, oder Carbamoyl bedeuten kann, zum Anilid I um.

Zur Darstellung von physiologisch verträglichen Säureadditionssalzen kann man die Anilide I mit üblicherweise verwendeten Salzbildnern (s. Arzneimittelforschung 10 (1966) 224, Birkhäuser Verlag) wie Chlorwasserstoffsäure, Bromwasserstoffsäure, Schwefelsäure, Phosphorsäure, Essigsäure, Malonsäure, Bernsteinsäure, Fumarsäure, Maleinsäure, Zitronensäure, Weinsäure, Milchsäure und Oxalsäure in an sich bekannter Weise umsetzen.

Die Verbindung I kann man in freier Form oder bevorzugt in Form eines Salzes mit einer physiologisch verträglichen Säure (s.o.) peroral, parenteral oder intravenös verabreichen.

Die Dosierung hängt vom Alter, Zustand und Gewicht des Patentien sowie von der Applikationsform ab. In der Regel beträgt die tägliche Wirkstoffdosis von 0,1 bis 20, vorzugsweise von 1 bis 10 mg/kg Körpergewicht bei oraler, und von 0,5 bis 5, vorzugsweise von 1 bis 3 mg/kg Körpergewicht bei intravenöser Anwendung.

Die Verbindungen I können in den gebräuchlichen galenischen Applikationsformen fest oder flüssig angewendet werden, z.B. als Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Emulsionen und Sprays.

Diese werden in üblicher Weise hergestellt. Die Wirkstoffe können dabei mit den gebräuchlichen galenischen Hilfsmitteln wie Tablettenbindern, Füllstoffen, Weichmachern, Netzmitteln, Dispergiermitteln, Emulgatoren, Lösungsmitteln, Retardierungsmitteln und/oder Antioxidantien verarbeitet werden (vgl. H. Sucker et al.: Pharmazeutische Technologie, Thieme Verlag, Stuttgart 1978). Die so erhaltenen Zubereitungen enthalten den Wirkstoff normalerweise in einer Menge von 0,1 bis 99 Gew.-%.

### Beispiele

### A) Synthesebeispiele

### Beispiel 1

### [3',5'-Dimethyl-4-(2-(N-pyrrolidinyl)ethoxy)]-(2-methylphenyl)-acetanilid-fumarat

Zu 2,3 g (10 mmol) 3,5-Dimethyl-4-(2-(N-pyrrolyl)ethoxyanilin in 100 ml Methylenchlorid wurden 15 ml einer 2 molaren Natronlauge gegeben. Anschließend wurde auf 0°C abgekühlt und mit 3,4 g (20 mmol) (2-Methylphenyl)essigsäurechlorid versetzt. Nach 12 h Rühren bei Raumtemperatur wurde die organische Phase abgetrennt und über Natriumsulfat getrocknet, wonach das Lösungsmittel im Vakuum entfernt wurde. Das Rohprodukt wurde in i-Propanol gelöst und mit 1,16 g (10 mmol) Fumarsäure versetzt, wobei 3,5 g des Säureadditionssalzes auskristallisierten. Schmp.: 164 bis 165°C.

### Beispiel 2

### [3', 5'-Dimethyl-4-(2-(N-pyrrolidinyl)ethoxy)]-(2-methylphenyl)-acrylsäureanilid-fumarat

Analog zu Beispiel 1 wurden 2,3 g (10 mmol) 3,5-Dimethyl-4-(2-N-pyrrolidinyl)ethoxy-anilin mit 3,6 g (20 mmol) des o-Methylzimtsäurechlorid umgesetzt. Es wurden 2,2 g kristallines Produkt erhalten. Schmp.: 200 bis 203°C.

### Beispiel 3

### [3', 5'-Dimethyl-4-(2-(N-pyrrolidinyl)ethoxy)]-3-(2-methylphenyl)-propionsäureanilid-fumarat

2,2 g des Produkts aus Beispiel 2 wurden in 100 ml Methanol gelöst und nach Zugabe von 0,25 g Pd/Kohle (10 Gew.-%) bei 25°C und einem Wasserstoffdruck von 1 bar hydriert. Anschließend wurde das Reaktionsgemisch filtriert und das Lösungsmittel im Vakuum entfernt. Das Rohprodukt wurde wie in Beispiel 1 in das Fumarat überführt und in Ethanol umkristallisiert, wobei 3,1 g Produkt erhalten wurden. Schmp.: 174 bis 176°C.

### B) Pharmakologische Wirkung

Die Wirkung der Anilide I als Repolarisationshemmstoffe kann man durch EKG-Messungen belegen. Dabei unterteilt man die Herzperiode zeitlich in Systole (Zusammenziehen des Herzens), auch QT-Dauer genannt, und Diastole (Erschlaffung des Herzens mit Blutfüllung der Herzkammern). Repolarisationshemmstoffe bewirken nun eine Verlängerung der QT-Dauer ohne die Vorhofkammer-Überleitungszeit (PQ-Dauer) und die Anspannungszeit (QRS-Dauer, Beginn der Systole bis zum öffnen der Semilunarklappen) wesentlich zu verändern (s. Pschyrembel, 254. Auflage, 1982).

Die Wirksamkeit der erfindungsgemäßen Verbindungen als Repolarisationshemmstoffe läßt sich im Tierversuch durch EKG-Messungen beispielsweise am Meerschweinchenherzen untersuchen (s. Basic Res. Cardiol. 82 (1987) 437; J. Pharmacol. Methods 21 (1989) 195). Zum Vergleich der Wirksamkeit mehrerer Substanzen dient dabei zum Beispiel die Dosis eines Wirkstoffes, bei der eine 20 %ige Zunahme des QT-Ausgangswertes eintritt (ED_{20 %} ). Hierzu trägt man die logarithmierten Dosiswerte der jeweiligen Substanzen gegen die experimentell gefundenen relativen Änderungen der QT-Dauer auf, und ermittelt mittels linearer Regression die Funktionsgleichung einer Geraden, aus der man dann den ED_{20 %}-Wert berechnen kann.

Mit dieser Methode wurden die ED_{20 %}-Werte der Verbindungen aus den Beispielen 2 und 3 bestimmt (s. Tabelle). Als Vergleichssubstanz diente D-Sotalol.

Als Versuchstiere dienten männliche Duncin-Hartley-Meerschweinchen mit einem Körpergewicht von 300 bis 350 g. 30 min nach Applikation von 1250 I.E. Heparin/kg Körpergewicht in den Bauchraum wurden die Tiere durch Genickschlag getötet. Nach dem Durchtrennen der Kopfschlagadern zum Entbluten, wurde der Bruststamm geöffnet, das Herz herausgetrennt und an eine Perfusionsapparatur angeschlossen. Die Perfusion erfolgte nach Langendorff mit Sauerstoff angereicherter auf 37°C erwärmter Krebs-Henseleit-Lösung (NaCl 6896 mg/l; KCI 350 mg/l; MgSO₄ 285 mg/l; CaCl₂ 370 mg/l; KH₂PO₄ 161 mg/l; NaHCO₃ 2090 mg/l; Glukose 2000 mg/l). Dabei wurde das Perfusionsvolumen pro Zeiteinheit bei einem Gesamtvolumen von 100 ml auf 4 bis 6 ml/min und der Perfusionsdruck auf 60 bis 70 mm Hg eingestellt. Nach einer Äquilibrierzeit von 30 min wurde zirkulierend perfundiert.

Die EKG-Messungen wurden über zwei Silberelektroden, die auf der Herzoberfläche im oberen Bereich der linken Koronararterie und auf der Rückseite des Herzens auf Höhe der Ventilebene angebracht waren, aufgenommen. Gemessen wurden die PQ-, QT- und QRS-Zeiten sowie die Herzfrequenz.

Die Substanzgabe erfolgte kumulativ im Abstand von 15 min ins Perfusat.

**Tabelle**

| Die QT-Zeit verlängernde Wirkung der erfindungsgemäßen Verbindungen 2 und 3 im Vergleich mit D-Sotalol | | |
|---|---|---|
| Beispiel Nr. | ED_{20 %} [µmol/l] | relative Wirkung ED_{20 %} (D-Sotalol)/ED_{20 %} (Bsp.) |
| 2 | 0,50 | 32 |
| 3 | 0,31 | 51 |
| | | |
| zum Vergleich: | | |
| | | |
| D-Sotalol | 16,00 | 1 |

## Patentansprüche

1. Substituierte Anilide der allgemeinen Formel I 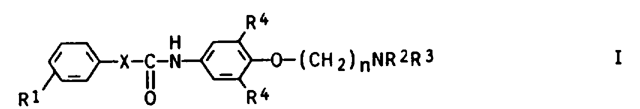 in der die Substituenten die folgende Bedeutung haben:
R¹ C₁-C₄-Alkyl, C₁-C₄-Halogenalkyl, C₁-C₄-Alkoxy, Halogen, Cyano, Nitro, C₂-C₄-Alkenyl, C₂-C₄-Alkinyl;
X Methylen, Ethylen, Vinylen;
R, R³ C₁-C₄-Alkyl, C₃-C₆-Cycloalkyl oder gemeinsam Tetra- oder Pentamethylen;
R⁴ C₁-C₄-Alkyl, Methoxy;
n 2, 3 oder 4,
sowie deren physiologisch verträgliche Säureadditionssalze.

2. Substituierte Anilide der allgemeinen Formel I gemäp Anspruch 1 zur Verwendung auf dem Arzneimittelgebiet.

3. Arzneimittel, enthaltend eine bei Herzrhythmusstörungen und/oder zur Herzfrequenzsenkung therapeutisch wirksame Menge der Verbindung I gemäß Anspruch 1.

4. Verwendung der Verbindung I und deren Salze mit physiologisch verträglichen Säuren gemäß Anspruch 1 zur Herstellung von Arzneimitteln zur Behandlung von Herzrhythmusstörungen und/oder zur Herzfrequenzsenkung.

## Claims

1. A substituted anilide of the formula I 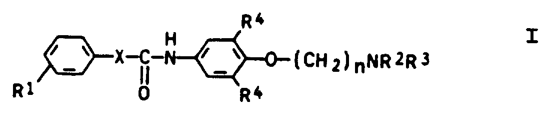 where
R¹ is C₁-C₄-alkyl, C₁-C₄-haloalkyl, C₁-C₄-alkoxy, halogen, cyano, nitro, C₂-C₄-alkenyl, C₂-C₄-alkynyl;
X is methylene, ethylene, vinylene;
R and R³ are each C₁-C₄-alkyl, C₃-C₆-cycloalkyl or together are tetra- or pentamethylene;
R⁴ is C₁-C₄-alkyl, methoxy;
n is 2, 3 or 4,
and the physiologically tolerated acid addition salts thereof.

2. A substituted anilide of the formula I as claimed in claim 1 for pharmaceutical use.

3. A drug containing an amount of the compound I as claimed in claim 1 which is therapeutically effective for cardiac arrhythmias and/or for lowering the heart rate.

4. The use of the compound I and the salts thereof with physiologically tolerated acids as claimed in claim 1 for the production of drugs for treating cardiac arrhythmias and/or for lowering the heart rate.

## Revendications

1. Anilides substitués de formule générale I 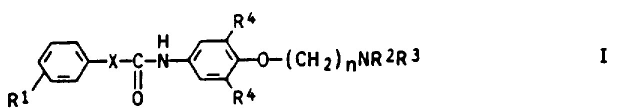 dans laquelle les substituants ont les significations suivantes:
R¹ Groupement alkyle en C₁-C₄, halogénoalkyle en C₁-C₄, alcoxy en C₁-C₄, atome d'halogène, groupement cyano, nitro, alcényle en C₂-C₄, alcynyle en C₂-C₄;
X Groupement méthylène, éthylène, vinylène;
R, R³ Groupements alkyle en C₁-C₄, cycloalkyle en C₃-C₆ ou, en commun, groupement tétra- ou pentaméthylène;
R⁴ Groupement alkyle en C₁-C₄, méthoxy;
n 2, 3 ou 4,
ainsi que leurs sels d'addition d'acide acceptables physiologiquement.

2. Anilides substitués de formule générale I selon la revendication 1, destinés à être utilisés dans le domaine des médicaments.

3. Médicament, contenant le composé I selon la revendication dans une quantité thérapeutiquement efficace pour les troubles du rythme cardiaque et/ou pour abaisser la fréquence cardiaque.

4. Utilisation du composé I ou de ses sels avec des acides acceptables physiologiquement selon la revendication 1 pour la fabrication de médicaments pour le traitement de troubles du rythme cardiaque et/ou pour abaisser la fréquence cardiaque.
